# FASCICULE DE BREVET EUROPEEN

(11) **EP 0 566 468 B1**
(45) Date de publication et mention de la délivrance du brevet: **22.01.1997**
(21) Numéro de dépôt: 93400948.1
(22) Date de dépôt: 09.04.1993
(51) Int. Cl.: C07C 253/30, C07C 255/50

(54) **Procédé de préparation d'un dérivé de biphényl**
Verfahren zur Herstellung eines Biphenylderivates
Process for the preparation of a biphenyl derivative

(30) Priorité: 13.04.1992 FR 9204512
(43) Date de publication de la demande: 20.10.1993
(73) Titulaire: SANOFI, 75008 Paris (FR)
(72) Inventeur: Bouisset, Michel, F-04200 Sisteron (FR); Boudin, Alain, F-04200 Sisteron (FR)
(74) Mandataire: Le Guen, Gérard

(56) Documents cités:
- EP-A- 0 341 514
- EP-A- 0 470 794
- CHEMICAL ABSTRACTS, vol. 75, no. 7, 1971, Columbus, Ohio, US; abstract no. 48572p, T. NAKAYA et al, "Coupling reaction of phenylmagnesium bromide with manganese(III) or cobalt(III)"

## Description

la présente invention se rapporte, d'une manière générale, à un nouveau procédé de préparation d'un dérivé de biphényle.

Plus précisément, l'invention a pour objet un nouveau procédé de préparation du méthyl-4 cyano-2' biphényle de formule :

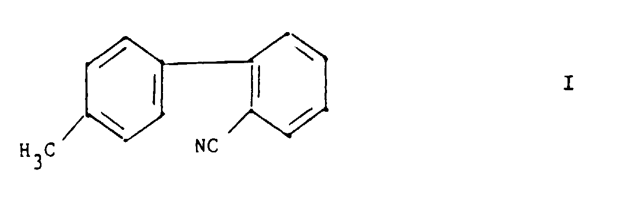

Le méthyl-4 cyano-2' biphényle peut être largement utilisé comme produit intermédiaire, notamment pour la synthèse finale de dérivés de biphénylméthylimidazoline décrits dans les demandes de brevet EP-A-0 253 310 et 0 454 511.

Ces dérivés d'imidazoline se sont révélés utiles en raison de leurs propriétés pharmacologiques, notamment leurs propriétés antagonistes de l'angiotensine II.

Ces propriétés rendent les composés en question particulièrement intéressants dans le traitement de syndromes du système cardiovasculaire comme l'hypertension, la défaillance cardiaque ainsi que dans le traitement d'affections du système nerveux central et dans le traitement du glaucome et de la rétinopathie diabétique.

On a rapporté dans la littérature chimique diverses méthodes permettant la préparation du méthyl-4 cyano-2' biphényle.

A cet égard, on peut citer le procédé décrit dans J. Med. Chem. 34 pp. 2525-2547 (1991), procédé selon lequel :
a) on fait réagir l'acide méthoxy-2 benzoïque avec le chlorure de thionyle ;
b) on traite le chlorure d'acyle formé avec l'amino-2 méthyl-2 propanol-1, ce qui fournit un amide sous forme brute ;
c) on soumet cet amide à l'action du chlorure de thionyle et on forme ainsi la diméthyl-4,4 (méthoxy-2 phényl)-2 oxazoline (rendement : 88% à partir du chlorure d'acyle) ;
d) on fait réagir ce dérivé d'oxazoline avec le bromure de p-tolylmagnésium et on hydrolyse le complexe formé, ce qui donne la diméthyl-4,4 (méthyl-4' biphénylyl-2)-2 oxazoline (rendement 91%) ;
e) on traite alors le dérivé d'oxazoline formé avec l'oxychlorure de phosphore, ce qui fournit finalement le méthyl-4 cyano-2' biphényle (rendement 96%).

En conséquence, le composé de formule I peut être synthétisé avec rendement global de 77% selon cette méthode. Néanmoins, ce procédé présente notamment le désavantage de nécessiter la mise en oeuvre de 5 étapes, au départ de produits commercialement disponibles, en raison de l'élaboration du groupement diméthoxyoxazolinyle intermédiaire et sa transformation ultérieure en groupement cyano.

En outre, la formation du chlorure de méthoxy-2 benzoyle requiert un traitement prolongé (18 heures) au moyen du chlorure de thionyle.

Par ailleurs, on a rapporté dans la demande de brevet EP-A-0 470 794 un procédé permettant la préparation de dérivés cyanobiphényles, notamment de dérivés méthyl-4 cyanobiphényles, procédé selon lequel on fait réagir un dérivé métallique ou organométallique de méthyl-4 phényle avec un bromobenzonitrile en présence d'un catalyseur métallique choisi parmi un catalyseur à base de Pd(O), Pd(II), Ni(O) et Ni(II).

Selon l'Exemple 2 de cette demande de brevet, on obtient le méthyl-4 cyano-2' biphényle par réaction du p-tolyltributylétain et du bromo-2 benzonitrile en présence de tétrakis (triphénylphosphine) palladium (O) avec un rendement de 63%. Cependant, ce procédé présente notamment le désavantage de nécessiter un temps de réaction très prolongé (36 heures) et la formation supplémentaire du p-tolyltribulétain à partir du bromure de p-tolylmagnésium.

La recherche d'un procédé industriel pour la préparation du méthyl-4 cyano-2' biphényle mettant en oeuvre un minimum d'étapes, des intermédiaires d'accès facile et peu onéreux, ainsi qu'un rendement satisfaisant en produit final reste d'un intérêt incontestable.

On a maintenant trouvé, de manière surprenante, qu'il est possible de préparer le méthyl-4 cyano-2' biphényle également au départ d'un halogénure de p-tolylmagnésium, mais selon un procédé industriel, en une seule étape, fournissant des rendements importants en composé désiré.

Ainsi, le procédé de l'invention pour la préparation du composé de formule I consiste à faire réagir, en présence d'un sel manganeux, un halogénure de benzonitrile de formule générale : 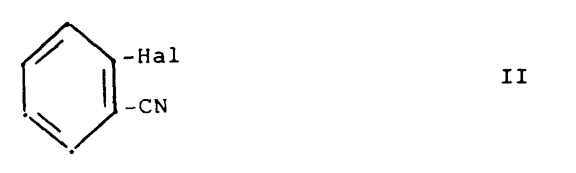 dans laquelle Hal représente un atome d'halogène, de préférence chlore, avec un dérivé organométallique, à savoir un dérivé magnésien de formule générale : 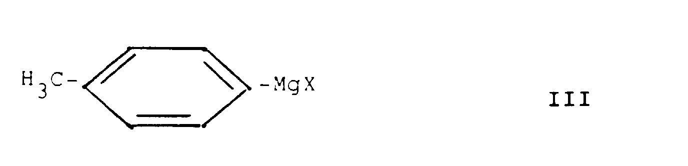 dans laquelle X représente un atome d'halogène, préférentiellement brome, puis à hydrolyser le complexe obtenu, ce qui fournit le composé désiré.

La réaction peut être effectuée selon des modes de mise en oeuvre classique de dérivés organométalliques, à savoir dans un éther anhydre tel que le tétrahydrofuranne, le dibutyl éther ou le dioxanne, et à une température comprise par exemple entre -10°C et la température ambiante.

De même, l'hydrolyse du complexe peut être effectuée selon des procédures connues par exemple au moyen d'un acide tel que l'acide chlorhydrique, en solution aqueuse. Généralement, on utilise le dérivé organométallique de formule III à raison de 1,5 à 2,5 moles par mole de dérivé de benzonitrile de formule II, de préférence 2 moles de composé de formule III.

Quant au sel manganeux, il peut s'agir notamment d'un halogénure manganeux, par exemple le chlorure, le bromure ou l'iodure.

Préférentiellement, on utilise le chlorure manganeux, étant donné son accessibilité commerciale et son coût peu élevé. Ce sel de manganèse peut être utilisé à raison de 0,05 à 1 mole par mole d'halogénure de benzonitrile de formule II.

On a en effet remarqué que de faibles concentrations, voire des concentrations quasi catalytiques de sel manganeux, telles que 5 à 10% molaire, peuvent être valablement utilisées pour la mise en oeuvre du procédé de l'invention.

L'utilisation de très faibles quantités de sel manganeux présente un avantage, notamment lors de la purification du composé de formule I. En effet, les sels de manganèse résiduels étant peu solubles dans le milieu réactionnel, leur présence en très faibles quantités, dans ce milieu, rendra leur séparation plus facile par décantation.

Selon une mise en oeuvre préférée du procédé de l'invention, on ajoute le dérivé organométallique de formule III à un mélange de composé de formule II et de sel manganeux dans un éther.

Toutefois, on peut également envisager d'ajouter le dérivé de benzonitrile de formule II à une solution de sel manganeux et de dérivé organométallique de formule III dans un éther.

Des essais préliminaires effectués dans le cadre de la présente invention ont montré que le bromure de p-tolylmagnésium, en présence de chloro-2 benzonitrile, mais en l'absence de sel de manganèse, se révèle incapable de donner accès au méthyl-4 cyano-2- biphényle.

Par exemple, on a réalisé un essai en ajoutant, à la température de 10°C ± 2°C et pendant 1 h 20 minutes, 70 ml d'une solution de bromure de p-tolylmagnésium (0,085 mole ; 1,22 équivalent) dans le tétrahydrofuranne à une solution de 9,6 g (0,07 mole) de chloro-2 benzonitrile dans 20 ml de tétrahydrofuranne et en maintenant le milieu à cette température durant 3 h 10 minutes.

Après hydrolyse au moyen d'acide chlorhydrique à 3,7% et extraction à l'acétate d'éthyle, une analyse par chromatographie liquide haute pression (CLHP) a montré la prépondérance du chloro-2 benzonitrile dans le milieu, ainsi que du produit d'addition du dérivé magnésien sur le nitrile, à savoir la (chloro-2 phényl-1) (tolyl-4) cétone.

Toutefois, on n'a observé aucune trace de méthyl-4 cyano-2' biphényle.

Par ailleurs, on a réalisé des essais comparatifs selon les conditions opératoires signalées dans la demande de brevet EP-A-0 470 794, et notamment selon les conditions opératoires de l'Exemple 2 de cette demande de brevet.

A cet effet, on a utilisé le procédé ci-dessous :

Dans un ballon tricol, on a réalisé sous atmosphère inerte une solution de 1 équivalent de bromo-2 benzonitrile et Y équivalent de catalyseur dans le tétrahydrofuranne.

On a alors coulé, à la température T, X équivalent de bromure de p-tolylmagnésium dénommé ci-après A-Mg Br, ou de p-tolyltributylétain, dénommé ci-après A-Sn Bu₃. En fin de coulée, on a maintenu le milieu réactionnel à la même température durant un temps t. On a ensuite hydrolysé avec une solution d'acide chlorhydrique à 5% et enfin avec de l'eau. On a extrait la phase aqueuse avec du toluène, on a réuni les phases organiques et on les a lavées avec de l'eau, avec une solution aqueuse à 5% de carbonate de potassium et enfin avec de l'eau.

On a ensuite déterminé le rendement en méthyl-4 cyano-2' biphényle, par titrage de ce composé dans le résidu de mise à sec de la phase organique.

On a obtenu les résultats suivants en utilisant comme catalyseur PdCl₂, NiCl₂ ou Pd (PPh₃)₄, Ph représentant le radical phényle :

| **X (équivalent)** | **Y (équivalent)** | **T°C/t (h)** | **Rendement (%)** |
|---|---|---|---|
| 4,2 A-MgBr | 0,3 PdCl₂ | 0/4 | 22 |
| 3,0 A-MgBr | 0,3 NiCl₂ | 0/4 | 27 |
| 1,0 A-SnBu₃ | 0,003 PdCl₂ | 65/14 | 1 |
| 1,0 A-SnBu₃ | 0,3 PdCl₂ | 65/20 | 6 |
| 1,0 A-SnBu₃ | 0,003 NiCl₂ | 65/14 | 0 |
| 1,0 A-SnBu₃ | 0,3 NiCl₂ | 65/20 | 0 |
| 4,0 A-MgBr | 0,003 Pd(PPh₃)₄ | 0/5 | 1 |
| 2,2 A-MgBr | 0,003 Pd(PPh₃)₄ | 65/6 | 1 |

Des essais supplémentaires effectués selon les mêmes conditions de l'état de la technique, mais en utilisant un catalyseur selon l'invention, à savoir MnCl₂, a fourni les résultats suivants :

| **X (équivalent)** | **Y (équivalent)** | **T°C/t (h)** | **Rendement (%)** |
|---|---|---|---|
| 1,0 A-SnBu₃ | 0,3 MnCl₂ | 65/14 | 1 |
| 1,0 A-SnBu₃ | 0,003 MnCl₂ | 65-20 | 0 |

De même, on a pratiqué d'autres essais comparatifs par mise en oeuvre de conditions opératoires selon l'invention au départ d'un équivalent de chloro-2 benzonitrile et en utilisant, comme dérivé organométallique soit le bromure de p-tolylmagnésium, selon l'invention, soit le p-tolyllithium, dénommé ci-après A-Li, selon l'état de la technique.

Les résultats suivants ont été enregistrés :

| **Dérivé organométallique** | **Sel manganeux (équivalent)** | **T°C/t (h)** | **Rendement** |
|---|---|---|---|
| 2,0 A-Li | 0,3 MnCl₂ | 0/4 | 0 |
| 1,73 A-MgBr | 0,2 MnCl₂ | 10/0,25 | 70 |
| 1,74 A-MgBr | 0,1 MnCl₂ | 10/0,25 | 70 |

Ces résultats montrent la nette supériorité du procédé de l'invention sur les procédés antérieurs.

Comme cité précédemment, le composé de formule I peut donner accès à des dérivés de biphénylméthylimidazoline décrits notamment dans les demandes de brevet EP-A-0 253 310 et 0 454 511.

Selon les cas, on appliquera le schéma réactionnel ci-dessous dans l'ordre indiqué, ou dans l'ordre inverse, à savoir :
a) la substitution du groupement méthyle du composé de formule I selon les méthodes connues, par exemple par condensation, en milieu basique, au moyen d'un composé approprié, et ce après halogénation de ce groupement méthyle,
b) la transformation du groupement cyano du composé de formule I selon des procédés classiques tel que par exemple au moyen d'azide de tributyl étain ou d'azide de sodium pour former le groupe tétrazolyle.

Les exemples non limitatifs suivants illustrent le procédé de l'invention.

### EXEMPLE 1

### Préparation du méthyl-4 cyano-2' biphényle

Dans un ballon tricol, on place 9,6 g (0,07 moles) de chloro-2 benzonitrile, 0,44 g ou 5% molaire de chlorure manganeux anhydre et 20 ml de tétrahydrofuranne sec. On coule alors goutte à goutte, en maintenant la température à 10°C ± 2°C, 110 ml d'une solution de bromure de p-tolylmagnésium (0,135 mole ; 1,93 équivalent) dans le tétrahydrofuranne. L'addition nécessite 1,5 h environ. On maintient ensuite le mélange pendant 15 minutes à cette température, puis on hydrolyse à cette même température avec 100 ml d'acide chlorhydrique à 3,7%. On décante et extrait la phase aqueuse avec 100 ml d'acétate d'éthyle. Après concentration des phases organiques, on obtient 19,4 g d'un liquide visqueux brun titrant, en CLHP, 41% en produit désiré, ce qui correspond à un rendement chimique de 60%.

De cette manière, on obtient le méthyl-4 cyano-2' biphényle sous forme d'un solide beige après recristallisations dans l'éthanol.
P.F. : 47-49°C.

### EXEMPLE 2

### Préparation du méthyl-4 cyano-2' biphényle

On utilise le même procédé que celui de l'Exemple 1 au départ de 0, 88 g ou 10% molaire de chlorure manganeux et 100 ml de bromure de p-tolylmagnésium (0,122 mole ; 1,74 équivalent) dans le tétrahydrofuranne. Après concentration des phases organiques, on obtient 18,3g d'un liquide visqueux brun titrant, en CLHP, 51% en produit désiré, ce qui correspond à un rendement chimique de 70%.

Après recristallisations éthanoliques, on peut isoler le méthyl-4 cyano-2' biphényle sous forme d'un solide beige.

### EXEMPLE 3

### Préparation du méthyl-4 cyano-2' biphényle

On utilise le même procédé que celui de l'Exemple 1 au départ de 1,76 g ou 20% molaire de chlorure manganeux et 90 ml de bromure de p-tolylmagnésium (0,121 mole ; 1,73 équivalent) dans le tétrahydrofuranne. Après concentration des phases organiques, on obtient 16,3g d'un liquide visqueux brun titrant, en CLHP, 58% en produit désiré, ce qui correspond à un rendement chimique de 70%.

Après recristallisations éthanoliques, on peut isoler le méthyl-4 cyano-2' biphényle sous forme d'un solide beige.

### EXEMPLE 4

### Préparation du méthyl-4 cyano-2' biphényle

On utilise le même procédé que celui de l'Exemple 1 au départ de 8,8 g ou 100% molaire de chlorure manganeux et 110 ml de bromure de p-tolylmagnésium (0,123 mole ; 1,76 équivalent) dans le tétrahydrofuranne. Après concentration des phases organiques, on obtient 17,4g d'un liquide visqueux brun titrant, en CLHP, 58,5% en produit désiré, ce qui correspond à un rendement chimique de 75%.

Après recristallisations éthanoliques, on peut isoler le méthyl-4 cyano-2' biphényle sous forme d'un solide beige.

## Revendications

1. Procédé de préparation du méthyl-4 cyano-2' biphényle de formule : 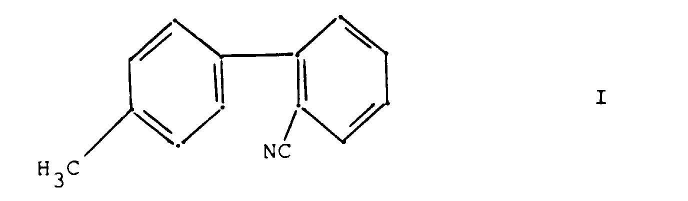 caractérisé en ce que l'on fait réagir, en présence d'un sel manganeux, un halogénure de benzonitrile de formule générale : 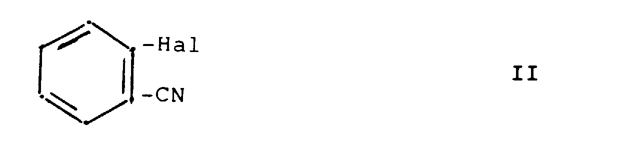 dans laquelle Hal représente un atome d'halogène, avec un dérivé organométallique de formule générale : 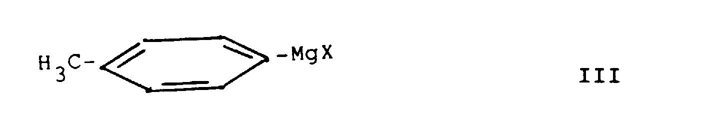 dans laquelle X représente un atome d'halogène, puis on hydrolyse le complexe obtenu, ce qui fournit le composé désiré.

2. Procédé selon la revendication 1, caractérisé en ce que Hal représente un atome de chlore.

3. Procédé selon la revendication 1 ou 2, caractérisé en ce que X représente un atome de brome.

4. Procédé selon l'une des revendications 1 à 3, caractérisé en ce que le sel manganeux est le chlorure manganeux.

5. Procédé selon l'une des revendications 1 à 3, caractérisé en ce que l'on utilise de 1,5 à 2,5 moles de dérivé organométallique de formule III par mole de dérivé de benzonitrile de formule II.

6. Procédé selon l'une des revendications 1 à 5, caractérisé en ce que l'on utilise de 0,05 à 1 mole de sel manganeux par mole de dérivé de benzonitrile de formule II.

7. Procédé selon l'une des revendications 1 à 6, caractérisé en ce que l'on hydrolyse le complexe au moyen d'un acide.

8. Procédé selon l'une des revendications 1 à 7, caractérisé en ce que l'on effectue la réaction à une température comprise entre -10°C et la température ambiante.

## Patentansprüche

1. Verfahren zur Herstellung von 4-Methyl-2'-cyano-biphenyl der Formel: 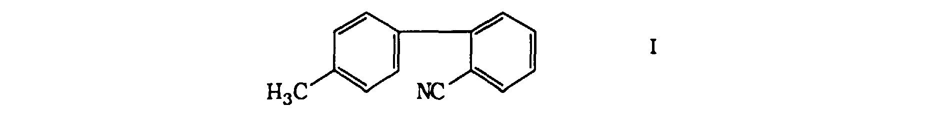 **dadurch gekennzeichnet**, daß man ein Benzonitrilhalogenid der allgemeinen Formel: 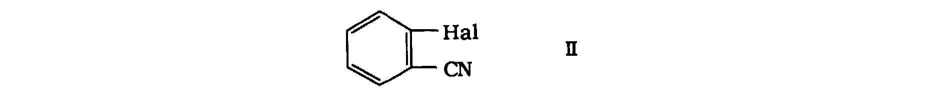 in der Hal ein Halogenatom bedeutet, in Gegenwart eines Mangan(II)-salzes mit einem metallorganischen Derivat der allgemeinen Formel: 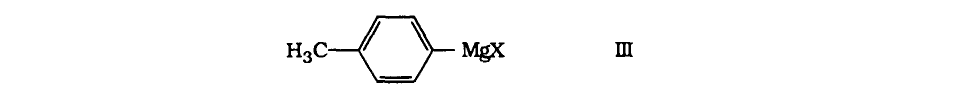 in der X ein Halogenatom bedeutet, umsetzt und den erhaltenen Komplex zur Bildung der gewünschten Verbindung hydrolysiert.

2. Verfahren nach Anspruch 1, **dadurch gekennzeichnet**, daß Hal ein Chloratom bedeutet.

3. Verfahren nach Anspruch 1 oder 2, **dadurch gekennzeichnet**, daß X ein Bromatom bedeutet.

4. Verfahren nach einem der Ansprüche 1 bis 3, **dadurch gekennzeichnet,** daß das Mangan(II)-salz Mangan(II)-chlorid ist.

5. Verfahren nach einem der Ansprüche 1 bis 3, **dadurch gekennzeichnet,** daß man 1,5 bis 2,5 Mol des metallorganischen Derivats der Formel III pro Mol des Benzonitrilderivats der Formel II verwendet.

6. Verfahren nach einem der Ansprüche 1 bis 5, **dadurch gekennzeichnet**, daß man 0.05 bis 1 Mol des Mangan(II)-salzes pro Mol des Benzonitrilderivats der Formel II verwendet.

7. Verfahren nach einem der Ansprüche 1 bis 6. **dadurch gekennzeichnet**, daß man den Komplex mit Hilfe einer Säure hydrolysiert.

8. Verfahren nach einem der Ansprüche 1 bis 7, **dadurch gekennzeichnet**, daß man die Reaktion bei einer Temperatur zwischen -10°C und Raumtemperatur durchführt.

## Claims

1. Process for preparing 4-methyl- 2'-cyano-biphenyl of formula: 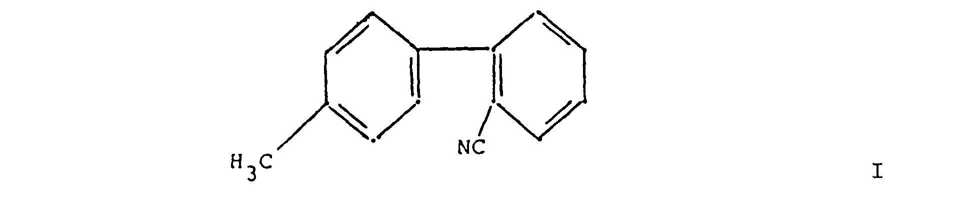 characterised in that a benzonitrile halide of general formula: 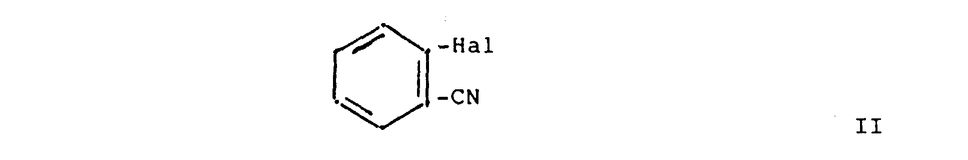 wherein Hal denotes a halogen atom, is reacted, in the presence of a manganese salt, with an organo metallic derivative of general formula: 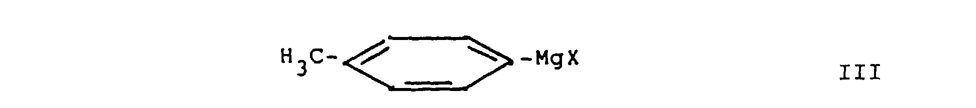 wherein X denotes a halogen atom, then the complex obtained is hydrolysed, to yield the desired compound.

2. Process according to claim 1, characterised in that Hal denotes a chlorine atom.

3. Process according to claim 1 or 2, characterised in that X denotes a bromine atom.

4. Process according to one of claims 1 to 3, characterised in that the manganese salt is manganese chloride.

5. Process according to one of claims 1 to 3, characterised in that 1.5 to 2.5 mols of organo metallic derivative of formula III are used per mol of benzonitrile derivative of formula II.

6. Process according to one of claims 1 to 5, characterised in that 0.05 to 1 mol of manganese salt are used per mol of benzonitrile derivative of formula II.

7. Process according to one of claims 1 to 6, characterised in that the complex is hydrolysed by means of an acid.

8. Process according to one of claims 1 to 7, characterised in that the reaction is carried out at a temperature between -10°C and ambient temperature.
